# EUROPÄISCHE PATENTSCHRIFT

(11) **EP 2 103 681 B1**
(45) Veröffentlichungstag und Bekanntmachung des Hinweises auf die Patenterteilung: **12.02.2014**
(21) Anmeldenummer: 09155621.7
(22) Anmeldetag: 19.03.2009
(51) Int. Cl.: C12M 1/107, C05F 17/00, C05F 17/02

(54) **Kombinierte Anlage zur Erzeugung von Biogas und Kompost sowie Verfahren zum Umschalten eines Fermenters in einer solchen Anlage zwischen Biogaserzeugung und Kompostierung**
Combined assembly for creating biogas and compost and method for adjusting a fermenter in such an assembly between biogas creation and composting
Installation combinée de production de biogaz et de compost et procédé de commutation d'un fermenteur dans une telle installation entre production de biogaz et compostage

(30) Priorität: 20.03.2008 DE 102008015240
(43) Veröffentlichungstag der Anmeldung: 23.09.2009
(73) Patentinhaber: Bekon Energy Technologies GmbH & CO. KG, 85774 Unterföhring (DE)
(72) Erfinder: Lutz, Peter, 81927 München (DE)
(74) Vertreter: Winter, Brandl, Fürniss, Hübner, Röss, Kaiser, Polte - Partnerschaft

(56) Entgegenhaltungen:
- EP-A1- 1 997 875
- EP-A2- 1 681 274
- DE-A1- 19 719 323

## Beschreibung

Die Erfindung betrifft eine kombinierte Anlage zur Erzeugung von Biogas und Kompost aus Biomasse mit wenigstens einem Fermenter nach dem Prinzip der Trockenfermentation gemäß Anspruch 1, sowie ein Verfahren zum Umschalten eines Fermenters in einer solchen Anlage zwischen Biogaserzeugung und Kompostierung gemäß Anspruch 13.

Die so genannte "Trockenfermentation" erlaubt es, schüttfähige Biomassen aus der Landwirtschaft, aus Bioabfällen und kommunalen Pflegeflächen zu methanisieren, ohne die Materialien in ein pumpfähiges, flüssiges Substrat zu überführen. Es können Biomassen mit bis zu 50% Trockensubstanzanteil vergoren werden. Dieses Trockenfermentationsverfahren ist beispielsweise in der EP 0 934 998 beschrieben.

Bei der "trockenen" Vergärung wird das zu vergärende Material nicht in eine flüssige Phase eingerührt, wie das zum Beispiel bei der Flüssigvergärung von Bioabfällen der Fall ist. Stattdessen wird das in den Fermenter eingebrachte Gärsubstrat ständig feucht gehalten, indem das Perkolat am Fermenterboden abgezogen und über der Biomasse wieder versprüht wird. So werden optimale Lebensbedingungen für die Bakterien erreicht. Bei der Rezirkulation des Perkolats kann zusätzlich die Temperatur reguliert werden, und es besteht die Möglichkeit. Zusatzstoffe für eine Prozessoptimierung zuzugeben.

Aus der WO 02/06439 ist ein Bioreaktor bzw. Fermenter in Form einer Fertiggarage bekannt, der nach dem Prinzip der Trockenfermentation im so genannten Batch-Verfahren betrieben wird. Hierbei wird nach einer Animpfung mit bereits vergorenem Material das Gärsubstrat mit Radladern in den Fermenter gefüllt. Der garagenförmig aufgebaute Gärbehälter wird mit einem gasdichten Tor verschlossen. Die Biomasse wird unter Luftabschluss vergoren, dabei erfolgt keine weitere Durchmischung und es wird kein zusätzliches Material zugeführt. Das aus dem Gärgut sickernde Perkolat wird über eine Drainagerinne abgezogen, in einem Tank zwischengespeichert und zur Befeuchtung wieder über dem Gärsubstrat versprüht. Der Gärprozess findet im mesophilen Temperaturbereich bei 34-37°C statt, die Temperierung erfolgt mittels einer Boden- und Wandheizung.

Das entstehende Biogas kann in einem Blockheizkraftwerk zur Gewinnung von Strom und Wärme genutzt werden. Damit immer genug Biogas für das Blockheizkraftwerk zur Verfügung steht, werden in der Trockenfermentationsanlage mehrere Gärbehälter zeitlich versetzt betrieben. Am Ende der Verweilzeit wird der Fermenterraum vollständig entleert und dann neu befüllt. Das vergorene Substrat wird anschließend einer Kompostierung zugeführt, so dass ein konventionellen Komposten vergleichbarer organischer Dünger entsteht.

Derartige Fermenter zur Erzeugung von Biogas nach dem Prinzip der Trockenfermentation sind auch aus der DE 203 19 847 U1 und der EP 1 681 274 A2 bekannt. Aus der DE 34 38 057 A1 ist es bekannt in einer Biogasanlage vergoren und verbrauchte Biomasse anschließend zu kompostieren.

Bedingt durch den Batch-Betrieb müssen die einzelnen Fermenter von Zeit zu Zeit, d. h. nachdem die in dem Fermenter befindliche Biomasse vollständig anaerob umgesetzt ist, abgeschaltet werden, d. h. die Biogasproduktion muss gestoppt, die vergorene Biomasse aus dem jeweiligen Fermenter entnommen, frische Biomasse in den Fermenter eingefüllt und die Biogasproduktion wieder aufgenommen werden. Nachteilig hierbei ist, dass aus sicherheitstechnischen Gründen verhindert werden muss, dass während des Ent- und Beladens der einzelnen Fermenter ein explosives Biogas/Luft-Gemisch entsteht.

Hierzu ist es aus der EP 1 301 583 B bekannt, einen laufenden Fermenter im Falle von Explosionsgefahr, d. h. wenn Luft in den Fermenter eingedrungen ist, mit kohlendioxidhaltigem Abgas aus dem mit Biogas betriebenen Blockheizkraftwerk zu fluten. Anschließend kann die vergorene Biomasse gefahrlos aus dem Fermenter entfernt und einer Kompostieranlage zugeführt werden. Aus der nachveröffentlichten EP 1 997 875 A1 ist eine herkömmliche Biogasanlage bekannt, bei der der Fermenter beim Be- und Entladen zunächst mit kohlendioxidhaltigem Abgas gespült wird.

Es ist daher Aufgabe der vorliegenden Erfindung eine Biogasanlage, wie sie aus der EP 1301583 B bekannt ist, derart weiter zu bilden, dass sich die Nachkompostierung der verbrauchten Biomasse vereinfacht.

Die Lösung dieser Aufgabe erfolgt durch die Merkmale der Ansprüche 1 und 12.

Dadurch, dass die verbrauchte Biomasse im Fermenter durch Umschalten von anaerober Vergärung auf aerobe Kompostierung kompostiert wird, erübrigt sich das Umsetzen der verbrauchten Biomasse in einen separaten Komposter. Die kombinierte Anlage nach Anspruch 1 umfasst die notwendigen Komponenten um ein sicheres Umschalten, Abschalten und Entladen und ebenso ein sicheres Anfahren eines Fermenters zu ermöglichen. Der erfindungsgemäße Fermenter ist so gestaltet, dass der gesamte Fermentierungsprozess, der sich aus einer anaeroben Vergärung und einer aeroben Kompostierung zusammensetzt, in ihm ablaufen kann, bevor eine Entnahme der verbrauchten Biomasse und eine erneute Beladung des Fermenters mit frischer Biomasse vorgenommen werden muss. Hierzu sind in der Bodenplatte des Fermenters Spülgaskanäle vorgesehen, die mit einem zweiten Spülgaseinlass verbunden sind.

Gemäß einer bevorzugten Ausgestaltung der Erfindung nach Anspruch 10 mündet im Bereich über der Biomasse in den Fermenter ein erster Spülgaseinlass.

Gemäß einer bevorzugten Ausgestaltung der Erfindung nach Anspruch 11 sind die Spülgaskanäle zur Abführung von aus der Biomasse während der Erzeugung von Biogas austretenden Sickerflüssigkeiten ausgelegt.

Durch die Maßnahmen nach Anspruch 12 wird die Biogasproduktion und - verwertung auch während des Beendens des Gärprozesses durch Spülen mit kohlendioxidhaltigem Abgas solange wie möglich aufrecht erhalten, d. h. das Biogas/Abgas-Gemisch des Fermenters wird solange weiter dem Biogasverbraucher zugeführt, bis die Qualität dieses Gemisches unter einen vorbestimmten Grad abnimmt, bevor dann der Fermenter zur Kompostierung der darin enthaltenen vergorenen Biomasse umgeschaltet wird. Erst wenn die Methankonzentration im Biogasauslass unter einen oberen Grenzwert absinkt, wird die zum Biogasverbraucher führende Biogasleitung von dem Biogasauslass getrennt. Danach wird das nur noch wenig Methan enthaltende Biogas/Abgas-Gemisch über einen Abluftkamin abgeführt. Dies erfolgt solange, bis die Methankonzentration auf einen unteren Grenzwert abgesunken ist, bei dem nahezu kein Methan mehr in dem Biogas/Abgas-Gemisch enthalten ist. Danach wird der Fermenter statt mit kohlendioxidhaltigem Abgas mit Frischluft gespült, und das Abgas/Biogas/Frischluft-Gemisch wird solange über den Abluftkamin abgeführt, bis die Kohlendioxidkonzentration in dem Abgas/Biogas/Frischluft-Gemisch auf einen ersten Grenzwert abgesunken ist. Erst dann wird der Fermenter zur Kompostierung umgeschaltet. Nach Beenden des Kompostierungsvorgangs kann der Fermenter geöffnet werden, um die verbrauchte Biomasse zu entladen und den Fermenter erneut mit frischer Biomasse zu beladen. Durch die sich an die Vergärung anschließende Kompostierung ist das Öffnen zum Ent- und erneuten Beladen des Fermenters gefahrlos möglich.

Gemäß einer bevorzugten Ausgestaltung der Erfindung nach Anspruch 13 wird das Biogas/Abgas-Gemisch bei Erreichen des oberen Grenzwertes der Methankonzentration nicht über den Abluftkamin an die Umwelt abgegeben, sondern einer Abgasfackel zugeführt und dort abgefackelt. Gegebenfalls kann die Abgasfackel mit zusätzlichem Brennstoff versorgt werden, so dass in jedem Fall eine Verbrennung statt findet. Das Abfackeln des Biogas/Abgas-Gemisches erfolgt solange, bis die Methankonzentration in dem Biogas/Abgas-Gemisch einen mittleren Grenzwert unterschreitet, der zwischen dem oberen und dem unteren Grenzwert liegt.

Gemäß bevorzugten Ausgestaltungen der Erfindung nach den Ansprüchen 14 und 16 wird der Kompostiervorgang durch Einstellen der Menge und/oder der Temperatur der über die Frischluftleitung zugeführten Frischluft gesteuert, um so ein optimales Prozessmilieu zu erreichen.

Gemäß einer bevorzugten Ausgestaltung der Erfindung nach Anspruch 16 werden die aus dem Fermenter abgeführten Gasgemische gefiltert. Durch die Filterung werden die Verbraucher möglicherweise schädigende Substanzen weitestgehend beseitigt, welche zum Beispiel zu Verstopfungen von Ventilen führen könnten.

Gemäß einer bevorzugten Ausgestaltung der Erfindung nach Anspruch 17 und 18 wird sicher verhindert, dass beim Anfahren ein explosives Biogas/Luft-Gemisch entsteht.

Das Aufschalten dieses wieder angefahrenen Fermenters auf die Biogasleitung erfolgt bei einem vierten Grenzwert der Methankonzentration, der gleich dem oberen Grenzwert ist - Anspruch 19.

Das Abgas zur Spülung des Fermenters wird beispielsweise von einer Verbrennungskraftmaschine bereit gestellt - Anspruch 20.

Gemäß einer bevorzugten Ausführungsform der Erfindung nach Anspruch 21 wird das kohlendioxidhaltige Abgas aus einer dem wenigstens einen Fermenter nachgeschalteten Biogasaufbereitungseinrichtung bereitgestellt.

Die übrigen Unteransprüche beziehen sich auf vorteilhafte Ausgestaltungen der Erfindung.

Weitere Einzelheiten, Merkmale und Vorteile der Erfindung zeigt die nachfolgende Beschreibung beispielhafter Ausführungsformen anhand der Zeichnungen:

Es zeigt:
Fign. 1 bis 7 schematische Darstellungen verschiedener Betriebszustände während des Beendens des Gärungsprozesses in einem Fermenter einer kombinierten Anlage und während des (wieder) Anfahrens des Fermenters;
Fig. 8 eine schematische Darstellung einer zweiten Ausführungsform der Erfindung mit einem Fermenter;
Fig. 9 bis 15 schematische Darstellungen verschiedener Betriebszustände einer kombinierten Anlage mit drei Fermentern während des Beendens des Gärungsprozesses in einem Fermenter einer kombinierten Anlage und während des (wieder) Anfahrens eines Fermenters;
Fig. 16 eine Fig. 1 entsprechende Darstellung einer Ausführungsform der Erfindung mit Abgas- bzw. Frischluftzufuhr aus der Bodenplatte des Fermenters;
Fig. 17A eine Aufsicht auf die Bodenplatte des Fermenters gemäß der Ausführungsform nach Fig. 16 mit Spülgaskanälen;
Fig. 17B eine Schnittdarstellung entlang der Line B - B in Fig. 17A mit Querkanal und den Spülgaskanälen; und
Fig. 17C eine Schnittdarstellung entlang der Line C - C in Fig. 17A mit den Spülgaskanälen.

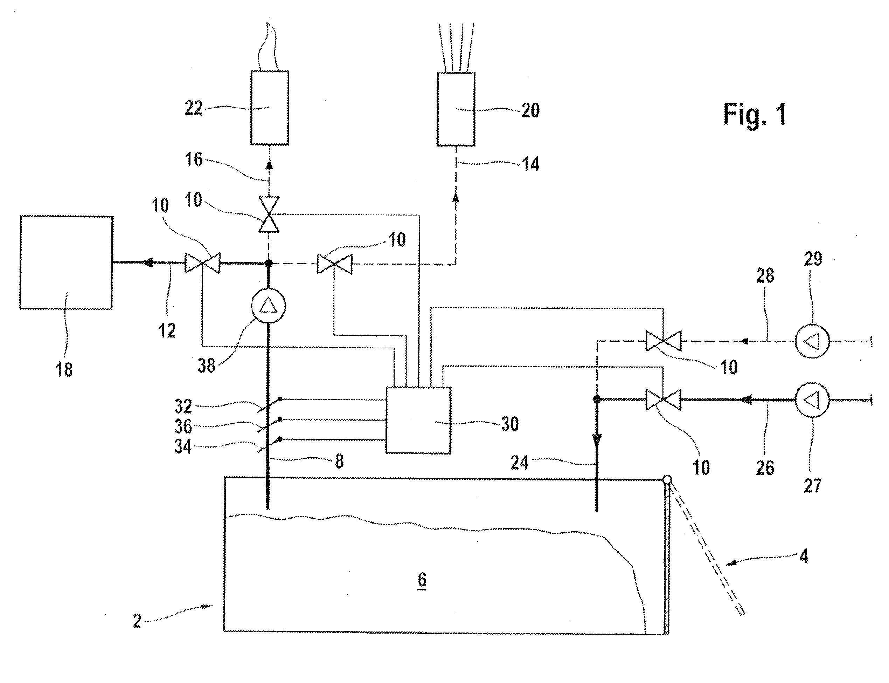

Die Figuren 1 bis 7 zeigen eine erste Ausführungsform einer kombinierten Anlage mit einem einzelnen Fermenter 2. Der Fermenter 2 ist quaderförmig und besitzt in etwa den Aufbau einer Fertiggarage. Über eine Be- und Entladeöffnung 4, die sich über eine der Stirnseiten des quaderförmigen Fermenters 2 erstreckt, lässt sich Biomasse 6 mittels eines Radladers in den Fermenter 2 einfüllen und wieder entnehmen. Hinsichtlich des genauen Aufbaus des Fermenters 2 wird auf die WO 02/06439 verwiesen.

Der Fermenter 2 umfasst weiter einen Biogasauslass 8, der über Ventile 10 mit einer Biogasleitung 12, einer ersten Biogas/Abgas-Leitung 14 und einer zweiten Biogas/Abgas-Leitung 16 verbindbar ist. Die Biogasleitung 12 führt zu einem Blockheizkraftwerk 18 als Biogasverwertungseinrichtung. Die erste Biogas/Abgas-Leitung 14 führt zu einem Bioabgaskamin 20. Die zweite Biogas/Abgas-Leitung 16 führt zu einer Abgasfackel 22. Weiter umfasst der Fermenter 2 einen Spülgaseinlass 24, der über Ventile 10 mit einer Abgasleitung 26 oder einer Frischluftleitung 28 verbindbar ist. In der Abgasleitung 26 ist ein Abgasgebläse 27 angeordnet, mittels dessen Abgas in den Fermenter 2 gepumpt werden kann. In der Frischluftleitung 28 ist ein Frischluftgebläse 29 zum Ansaugen von Frischluft aus der Umgebung angeordnet. Über die Abgasleitung 26 wird kohlendioxidhaltiges Abgas als Spülgas und über die Frischluftleitung 28 Frischluft in den Fermenter 2 geführt.

Die Ventile 10 sind mit einer Steuereinrichtung 30 verbunden und werden durch die Steuereinrichtung 30 geöffnet oder geschlossen. Die Steuereinrichtung 30 ist auch mit einem ersten Messfühler 32 verbunden, der im Biogasauslass 8 angeordnet ist und die Methankonzentration in dem jeweiligen Gasgemisch erfasst. Die Steuereinrichtung 30 ist weiter mit einem zweiten Messfühler 34 verbunden, der ebenfalls im Biogasauslass 8 angeordnet ist und die Kohlendioxidkonzentration in dem jeweiligen Gasgemisch erfasst. Die Steuereinrichtung 30 ist auch mit einem dritten Messfühler 36 verbunden, der im Biogasauslass 8 angeordnet ist und den Gasvolumenstrom in dem Biogasauslass erfasst. Über ein in dem Biogasauslass angeordnetes Gebläse 38 kann der Abzug von Gas aus dem Fermenter 2 gegebenenfalls unterstützt werden.

In den Figuren 1 bis 7 sind verschieden Phasen des Beendens des Gärungsprozesses in dem Fermenter 2 und des Anfahrens des Fermenters 2 dargestellt, wobei aktive Leitungen und Stellungen von Komponenten durchgezogen dargestellt sind, während nicht-aktive bzw. abgesperrte Leitungen und Stellungen von Komponenten strichliert dargestellt sind. Erfindungsgemäß folgt direkt im Anschluss an den Gärungsprozess die aerobe Umsetzung der vergorenen Biomasse in demselben Fermenter, in dem dieser in geeigneter Weise zur Kompostierung umgeschaltet wird, bevor dann der Fermenter geöffnet, entladen, neu beladen und wieder angefahren wird.

Fig. 1 zeigt die erste Phase des Beendens des Gärungsprozesses in dem Fermenter 2, in der kohlendioxidhaltiges Abgas über die Abgasleitung 26 und den Spülgaseinlass 24 in das Innere des Fermenters 2 gepumpt wird. Der Biogasauslass 8 ist nach wie vor mit der Biogasleitung 12 verbunden, so dass das Biogas/Abgas-Gemisch weiter dem BHKW 18 zugeführt wird.

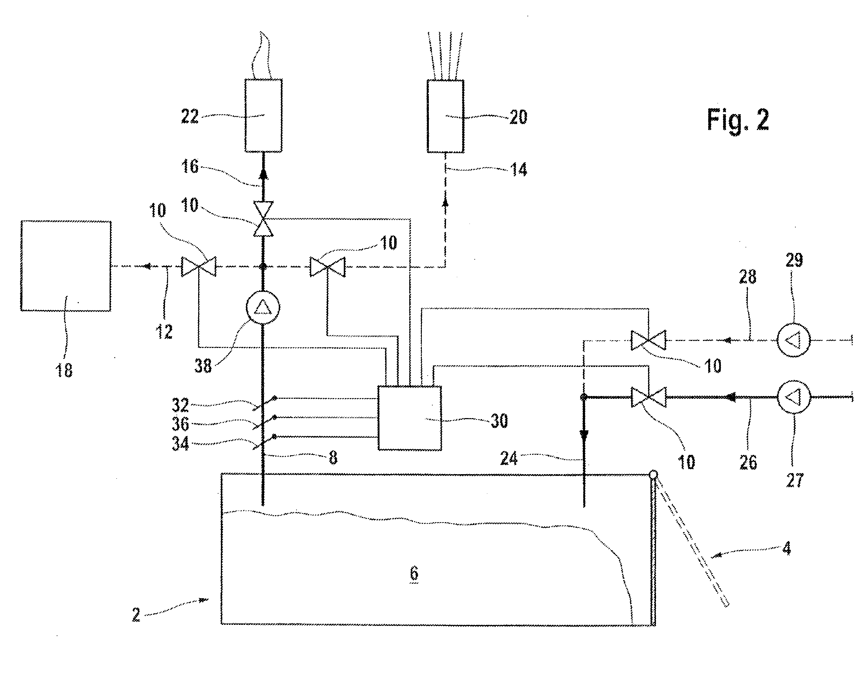

Erst wenn die durch den ersten Messfühler 32 in dem Biogasauslass 8 erfasste Methankonzentration unter einen oberen Grenzwert abgesunken ist, wird in einer zweiten Phase durch die Steuereinrichtung 30 das Ventil 10 in der Biogasleitung 12 geschlossen und das Ventil 10 in der zweiten Biogas/Abgas-Leitung 16 geöffnet, wie dies in Fig. 2 dargestellt ist. In dieser zweiten Phase des Beendens des Gärungsprozesses in dem Fermenter 2 wird das Biogas/Abgas-Gemisch in der Abgasfackel 22 verbrannt. Gegebenenfalls kann dieser Verbrennungsprozess durch Zugabe von zusätzlichem Brennstoff unterstützt werden.

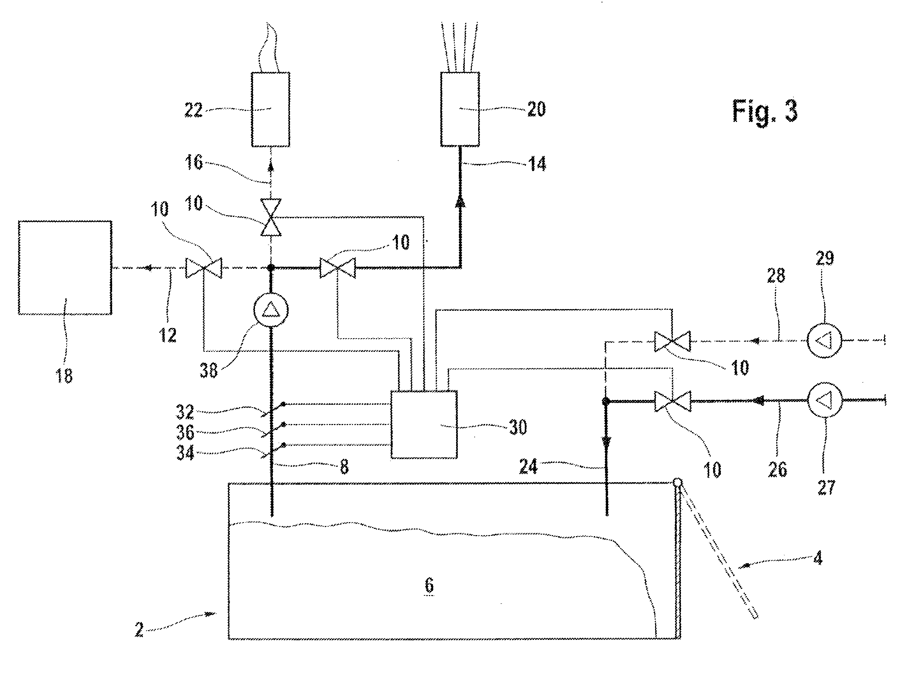

Wenn die durch den ersten Messfühler 32 in dem Biogasauslass 8 erfasste Methankonzentration unter einen mittleren Grenzwert abgesunken ist, wird in einer dritten Phase durch die Steuereinrichtung 30 das Ventil 10 in der zweiten Biogas/AbgasLeitung 16 geschlossen und das Ventil 10 in der ersten Biogas/Abgas-Leitung 14 geöffnet, wie dies in Fig. 3 dargestellt ist. In dieser dritten Phase des Beendens des Gärungsprozesses in dem Fermenter 2 wird das Biogas/Abgas-Gemisch über den Abgaskamin 20 an die Umgebung abgegeben.

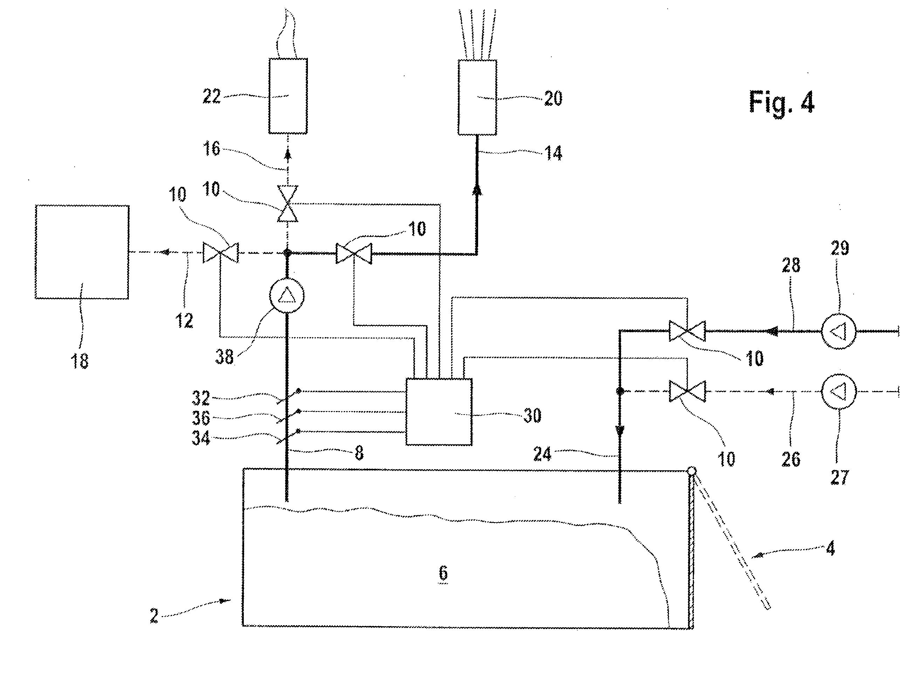

Wenn die durch den ersten Messfühler 32 in dem Biogasauslass 8 erfasste Methankonzentration unter einen unteren Grenzwert abgesunken ist, wird in einer vierten Phase durch die Steuereinrichtung 30 das Ventil 10 in der Abgasleitung 26 geschlossen und das Ventil 10 in der Frischluftleitung 28 geöffnet, wie dies in Fig. 4 dargestellt ist. In dieser vierten Phase des Beendens des Gärungsprozesses in dem Fermenter 2 wird über die Frischluftleitung 28 und den Spülgaseinlass 24 Frischluft in den Fermenter 2 gepumpt. Das Abgas/Luft-Gemisch wird weiter über den Biogasauslass 8 und die erste Biogas/Abgas-Leitung 14 in dem Abgaskamin 20 an die Umgebung abgegeben.

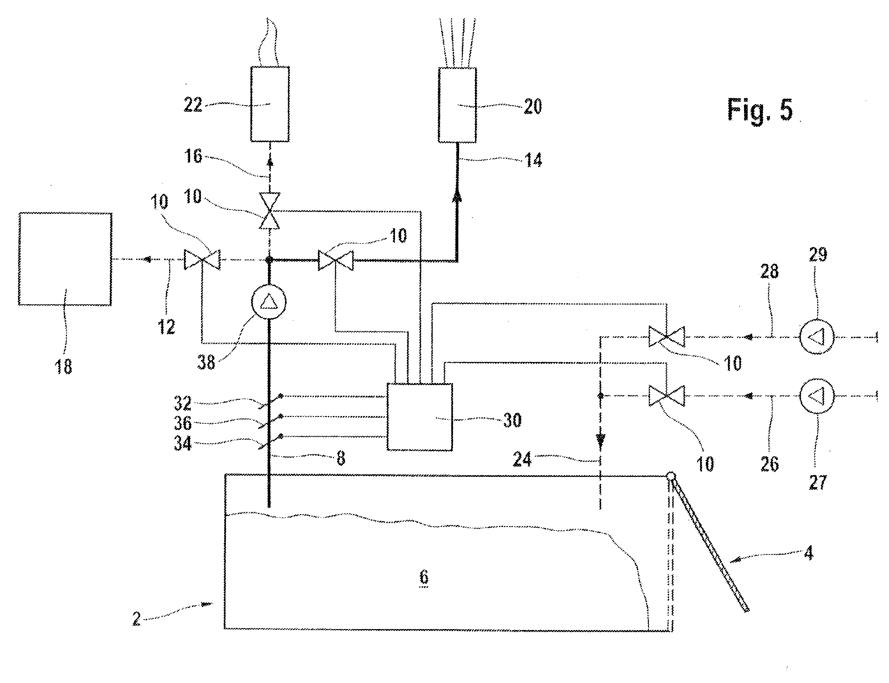

Wenn die durch den zweiten Messfühler 34 in dem Biogasauslass 8 erfasste Kohlendioxidkonzentration unter einen ersten Grenzwert abgesunken ist, wird der Fermenter auf aerobe Prozessführung umgeschaltet, so dass die sich im nach wie vor geschlossenen Fermenter befindliche, vergorene Biomasse kompostiert wird. Am Ende der Kompostierung wird das Ventil 10 in der Frischluftleitung 28 durch die Steuereinrichtung 30 geschlossen und die Be- und Entladeöffnung 4 geöffnet, wie dies in Fig. 5 dargestellt ist.

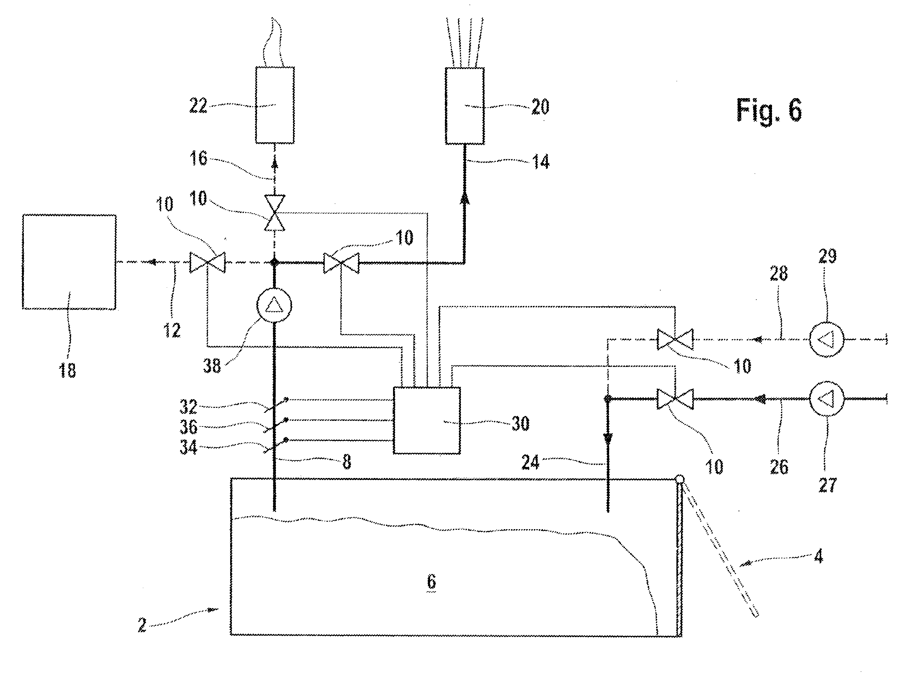

Ist der Fermenter 2 wieder mit frischer Biomasse beladen, wird die Be- und Entladeöffnung 4 geschlossen, die Verbindung zwischen Biogasauslass 8 und Abgaskamin 20 über die erste Biogas/Abgas-Leitung 14 wird aufrecht erhalten, und die Steuereinrichtung 30 öffnet das Ventil 10 in der Abgasleitung 26, so dass kohlendioxidhaltiges Abgas in den Fermenter 2 gepumpt wird - siehe Fig. 6. Dies wird solange fortgesetzt, bis die durch den zweiten Messfühler 34 erfasste Kohlendioxidkonzentration in dem Biogasauslass 8 einen zweiten Grenzwert erreicht bzw. übersteigt.

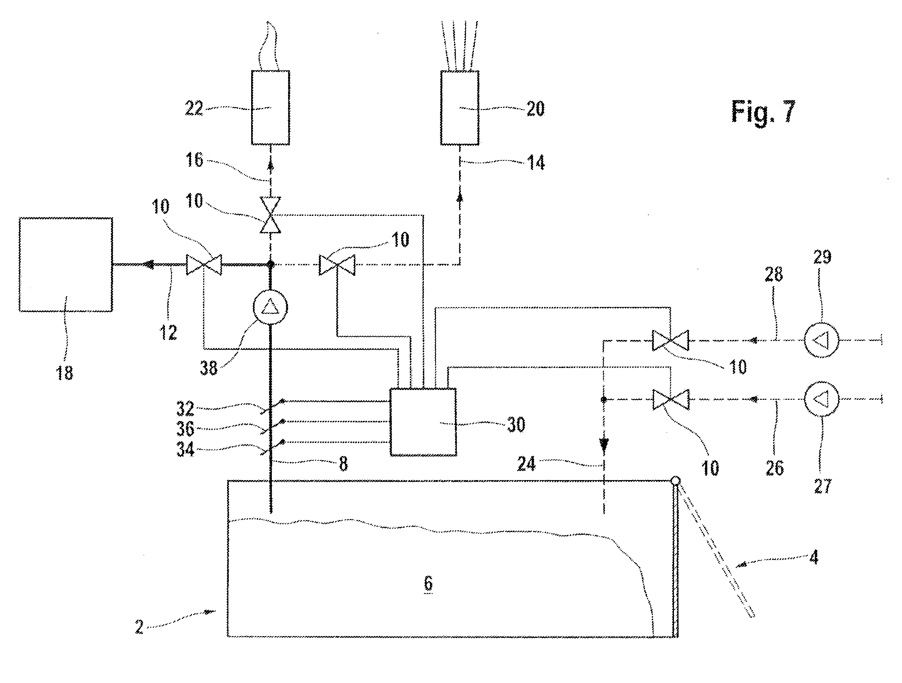

Ist dieser zweite Grenzwert für die Kohlendioxidkonzentration erreicht, wird durch die Steuereinrichtung 30 das Ventil 10 in der Abgasleitung 26 und in der ersten Biogas/Abgas-Leitung 14 geschlossen und das Ventil 10 in der Biogasleitung 12 geöffnet, wie dies in Fig. 7 dargestellt ist. Damit ist die Phase der Biogasproduktion wieder erreicht und das in dem Fermenter 2 erzeugte Biogas wird über die Biogasleitung 12 dem BHKW 18 zugeführt.

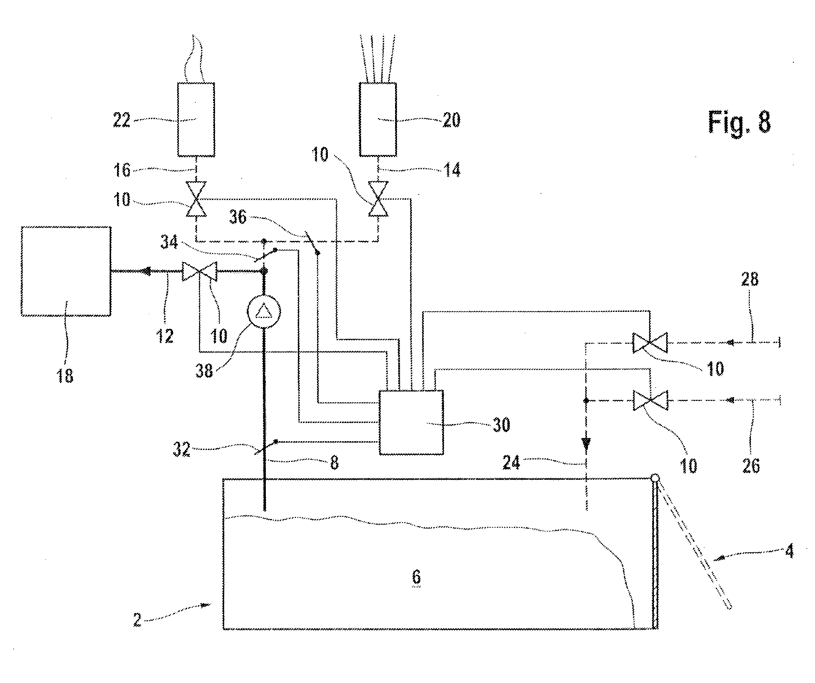

Bei der vorstehend beschriebenen Ausführungsform sind alle Messfühler 32, 34, 36 in dem Biogasauslass 8 angeordnet. Alternativ können gemäß einer zweiten Ausführungsform der vorliegenden Erfindung der zweite und dritte Messfühler 24, 36 auch in der ersten bzw. zweiten Biogas/Abgas-Leitung 14, 16 angeordnet sein. Fig. 8 zeigt eine alternative Ausgestaltung der Erfindung, die sich von der Ausführungsform nach den Fign. 1 bis 7 dadurch unterscheidet, dass die erste und zweite Biogas/Abgas-Leitung 14, 16 zu einer gemeinsamen Biogas/Abgas-Leitung 40 zusammengefasst sind, bevor sie in den Biogasauslass 8 münden. Der zweite Messfühler zur Erfassung der Kohlendioxidkonzentration ist in der gemeinsamen Biogas/AbgasLeitung 40 angeordnet, und der dritte Messfühler 36 ist in der ersten Biogas/AbgasLeitung 14 angeordnet. Ansonsten stimmt diese zweite Ausführungsform der Erfindung mit der ersten Ausführungsform überein. Auch die Funktionsweise ist identisch.

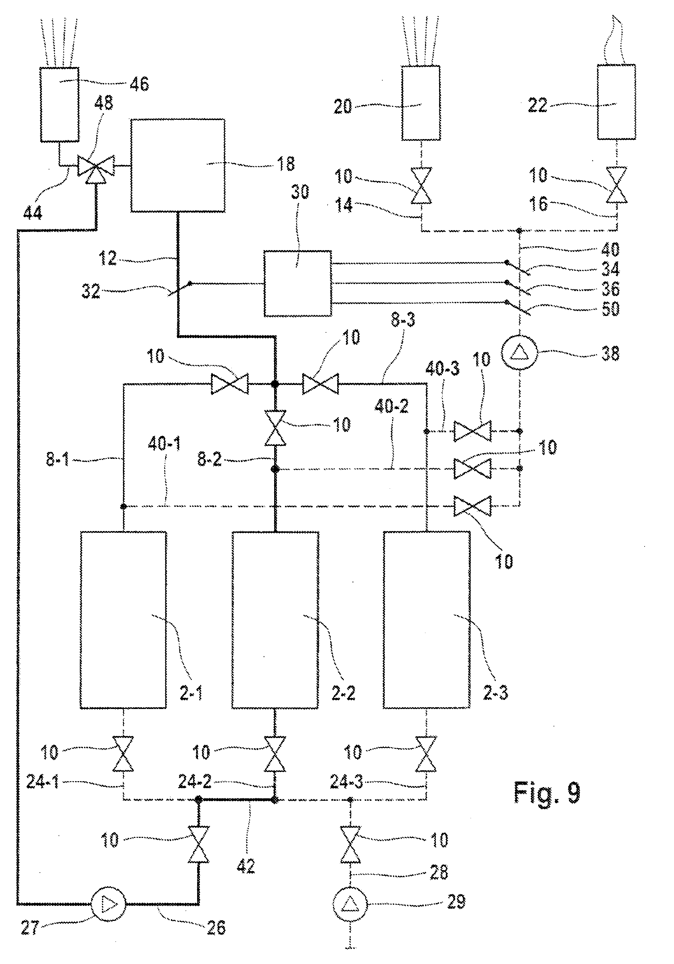

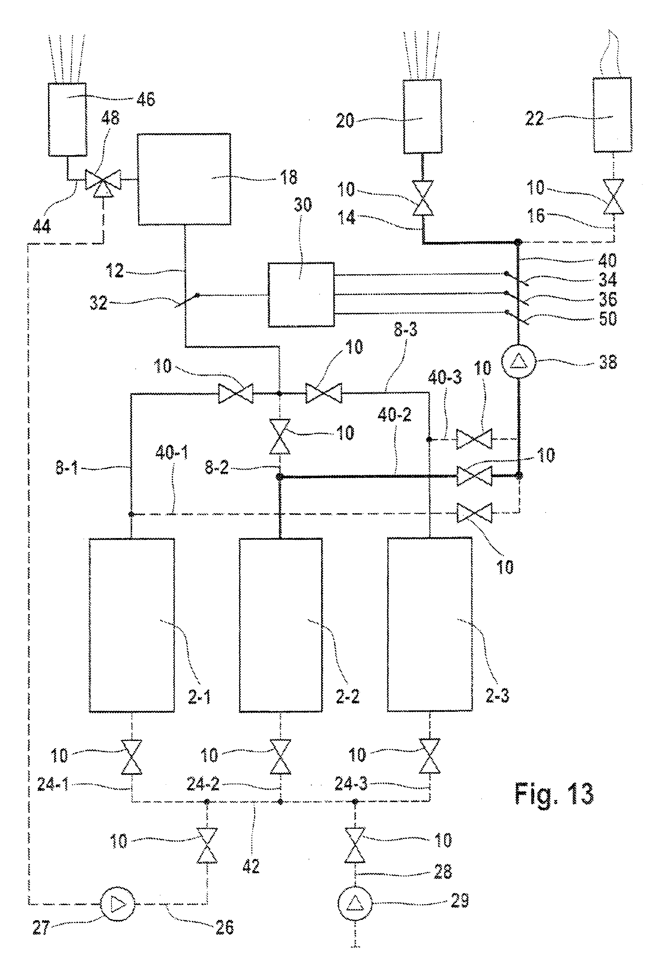

Die Figuren 9 bis 15 zeigen eine dritte Ausführungsform einer kombinierten Anlage, bei der drei Fermenter 2-1, 2-2 und 2-3 (im Folgenden zusammenfassend mit "2-i" bezeichnet) im Parallelbetrieb vorgesehen sind. Einander entsprechende Komponenten sind mit den gleichen Bezugszeichen versehen. Bei der kombinierten Anlage nach den Figuren 9 bis 15 ist jeder der drei Fermenter 2-i mit einem Spülgaseinlass 24-1, 24-2 bzw. 24-3 versehen, die jeweils mit einem Ventil 10 absperrbar sind. Die drei Spülgaseinlässe 24-i sind zu einem gemeinsamen Spülgaseinlass 42 zusammengefasst. In den gemeinsamen Spülgaseinlass 42 münden eine Abgasleitung 26 und eine Frischluftleitung 28, die jeweils durch ein Ventil 10 absperrbar sind .

Jeder der drei Fermenter 2-i ist mit einem Biogasauslass 8-1, 8-2 bzw. 8-3 versehen, die jeweils mit einem Ventil 10 absperrbar sind. Die erste Biogas/AbgasLeitung 14 zu dem Abgaskamin 20 und die zweite Biogas/Abgas-Leitung 16 zu der Abgasfackel 22 sind zu einer gemeinsamen Biogas/Abgas-Leitung 40 zusammengefasst, in der ein Gebläse 38 angeordnet ist. Nach dem Gebläse 38 spaltet sich die gemeinsame Biogas/Abgas-Leitung 40 in eine erste, eine zweite und eine dritte Teil-Biogas/Abgas-Leitung 40-1, 40-2 bzw. 40-3 auf. Die erste Teil-Biogas/Abgas-Leitung 40-1 mündet zwischen Ventil 10 und dem ersten Fermenter 2-1 in den ersten Biogasauslass 8-1. Die zweite Teil-Biogas/Abgas-Leitung 40-2 mündet zwischen Ventil 10 und dem zweiten Fermenter 2-2 in den zweiten Biogasauslass 8-2. Die dritte Teil-Biogas/Abgas-Leitung 40-3 mündet zwischen Ventil 10 und dem dritten Fermenter 2-3 in den dritten Biogasauslass 8-3. Die drei Teil-Biogas/Abgas-Leitungen 40-1, 40-2 und 40-3 sind jeweils durch ein Ventil 10 absperrbar. Die drei Biogasauslässe 8-1, 8-2 und 8-3 münden in eine gemeinsame Biogasleitung 12, die zu einem Blockheizkraftwerk 18 führt. Eine Auspuffleitung 44 aus dem BHKW 18 mündet einen zweiten Abgaskamin 46. Die Abgasleitung 26 ist über ein 3-Wege-Ventil 48 mit der Auspuffleitung 44 verbunden, d. h. das in dem BHKW 18 anfallende kohlendioxidhaltige Abgas wird zum Spülen eines Fermenters 2-i verwendet, dessen Gärprozess beendet und der in den Kompostierungsprozess umgeschaltet werden soll. Durch das 3-Wege-Ventil kann der Volumenstrom des Abgases, der zum Spülen eines Fermenters 2-i über die Abgasleitung 26 geschickt wird, und die Menge des Abgases, die über den zweiten Abgaskamin 46 an die Umgebung abgegeben wird, geregelt werden.

Ein erster Messfühler 32 ist zur Erfassung der Methankonzentration in der gemeinsamen Biogasleitung 12 angeordnet. Ein zweiter Messfühler 34 zur Erfassung der Kohlendioxidkonzentration, ein dritter Messfühler 36 zur Erfassung des Volumenstroms und ein vierter Messfühler 50 zur Erfassung der Methankonzentration sind in der gemeinsamen Biogas/Abgas-Leitung 40 in Strömungsrichtung nach dem Gebläse 38 angeordnet. Die vier Messfühler 32, 34, 36 und 50 sind mit einer Steuereinrichtung 30 verbunden. Ebenfalls mit der Steuereinrichtung verbunden sind die jeweiligen Ventile 10. Aus Gründen der Übersichtlichkeit sind die entsprechenden Steuerleitungen nicht in den Figuren 9 bis 15 eingezeichnet.

In den Figuren 9 bis 15 ist das Beenden des Gärungsprozesses in dem Fermenter 2-2 und das wieder Anfahren des zweiten Fermenters 2-2 nach dem Kompostierungsprozess, der sich direkt an den Gärungsprozess anschließt und durch Umschalten des Fermenters 2-2 eingeleitet wird, dargestellt, wobei die Figuren 9 bis 15 die gleichen Phasen und Betriebszustände wie die Figuren 1 bis 7 darstellen. Die Biogasproduktion des ersten und dritten Fermenters 2-1 bzw. 2-3 läuft während des Beendens des Gärungsprozesses und des Kompostierungsprozesses in dem zweiten Fermenter 2-2 und des wieder Anfahrens des zweiten Fermenters 2-2 kontinuierlich durch.

Fig. 9 zeigt die erste Phase des Beendens des Gärungsprozesses in dem Fermenter 2-2, in der kohlendioxidhaltiges Abgas aus dem BHKW 18 über das 3-Wege-Ventil 48 und die Abgasleitung 26, das Abgasgebläse 27 und den zweiten Spülgaseinlass 24-2 in das Innere des Fermenters 2-2 gepumpt wird. Der zweite Biogasauslass 8-2 ist nach wie vor mit der gemeinsamen Biogasleitung 12 verbunden, so dass das Biogas/Abgas-Gemisch weiter der Gasaufbereitungseinrichtung 44 zugeführt wird.

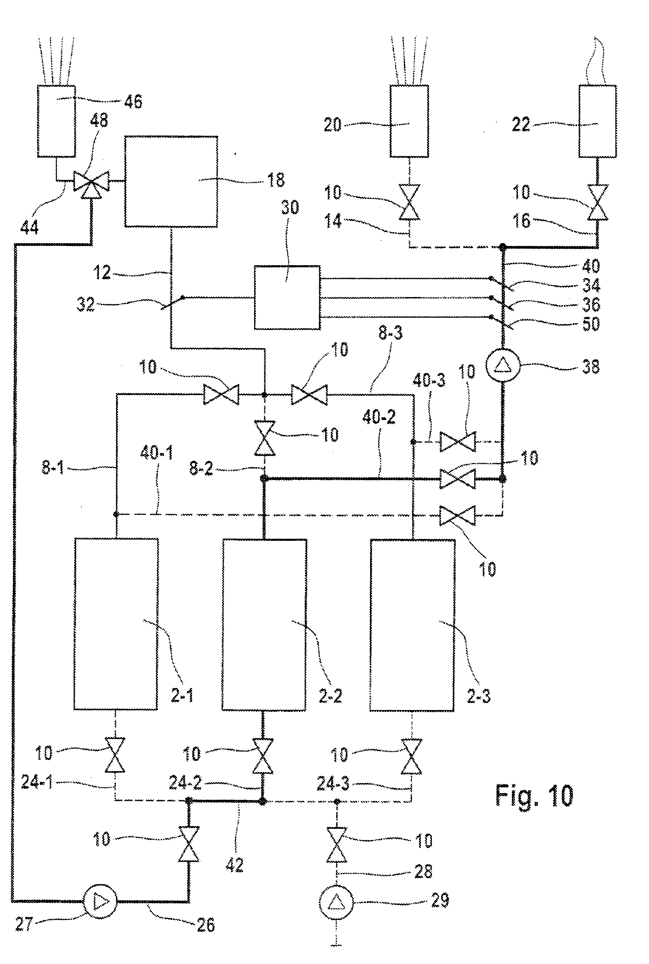

Erst wenn die durch den ersten Messfühler 32 in der gemeinsamen Biogasleitung 12 erfasste Methankonzentration unter einen oberen Grenzwert abgesunken ist, wird in einer zweiten Phase durch die Steuereinrichtung 30 das Ventil 10 im zweiten Biogasauslass 8-2 geschlossen und das Ventil 10 in der zweiten Teil-Biogas/AbgasLeitung 40-2 und in der zweiten Biogas/Abgas-Leitung 16 geöffnet, wie dies in Fig. 10 dargestellt ist. In dieser zweiten Phase des Beendens des Gärungsprozesses in dem Fermenter 2-2 wird das Biogas/Abgas-Gemisch in der Abgasfackel 22 verbrannt. Gegebenenfalls kann dieser Verbrennungsprozess durch Zugabe von zusätzlichem Brennstoff unterstützt werden.

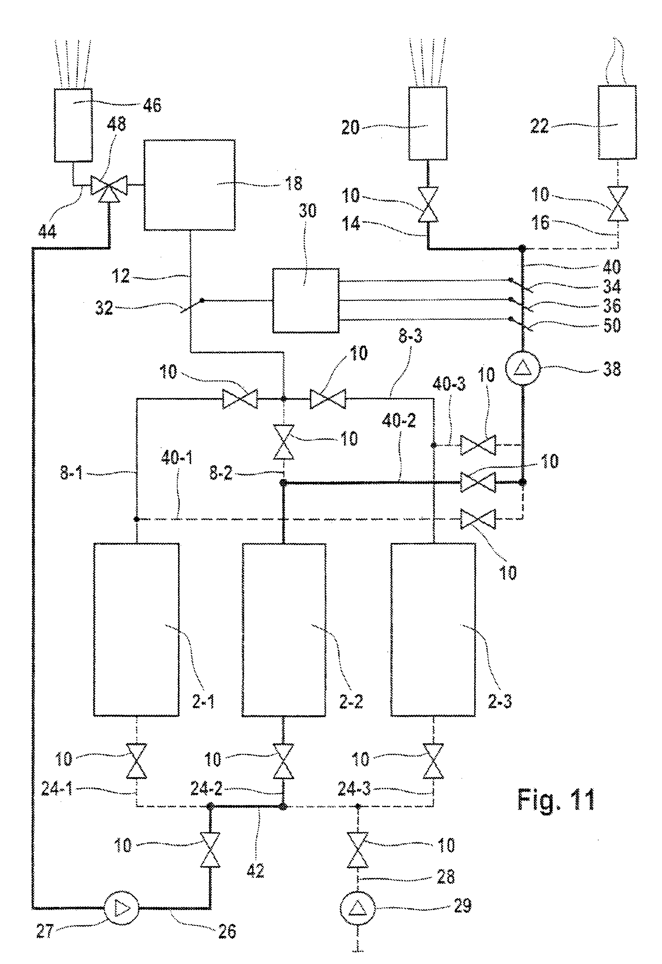

Wenn die durch den vierten Messfühler 50 in der gemeinsamen Biogas/AbgasLeitung 40 erfasste Methankonzentration unter einen mittleren Grenzwert abgesunken ist, wird in einer dritten Phase durch die Steuereinrichtung 30 das Ventil 10 in der zweiten Biogas/Abgas-Leitung 16 geschlossen und das Ventil 10 in der ersten Biogas/Abgas-Leitung 14 geöffnet, wie dies in Fig. 11 dargestellt ist. In dieser dritten Phase des Beendens des Gärungsprozesses in dem Fermenter 2-2 wird das Biogas/Abgas-Gemisch über den Abgaskamin 20 an die Umgebung abgegeben.

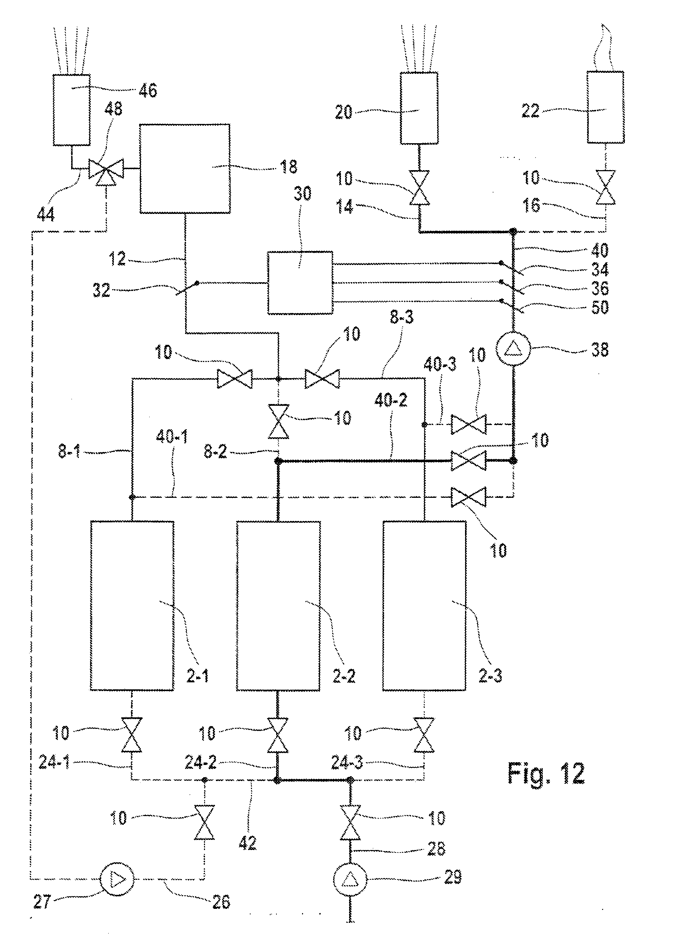

Wenn die durch den vierten Messfühler 50 in der gemeinsamen Biogas/AbgasLeitung 40 erfasste Methankonzentration unter einen unteren Grenzwert abgesunken ist, wird in einer vierten Phase durch die Steuereinrichtung 30 das Ventil 10 in der Abgasleitung 26 geschlossen, das 3-Wege-Ventil 48 entsprechend geschaltet und das Ventil 10 in der Frischluftleitung 28 geöffnet, wie dies in Fig. 12 dargestellt ist. In dieser vierten Phase des Beendens des Gärungsprozesses in dem Fermenter 2-2 wird über die Frischluftleitung 28 und den Spülgaseinlass 24 durch das Frischluftgebläse 29 Frischluft in den Fermenter 2-2 gepumpt. Das Abgas/Luft-Gemisch wird weiter über den zweiten Biogasauslass 8-2, die zweite Teil-Biogas/Abgas-Leitung 40-2, die gemeinsame Biogas/Abgas-Leitung 40 und die erste Biogas/Abgas-Leitung 14 in dem Abgaskamin 20 an die Umgebung abgegeben. Gegebenenfalls kann dies durch das Gebläse 38 unterstützt werden.

Wenn die durch den zweiten Messfühler 34 in der gemeinsamen Biogasleitung 40 erfasste Kohlendioxidkonzentration unter einen ersten Grenzwert abgesunken ist, wird der Fermenter 2-2 umgeschaltet und die Kompostierung eingeleitet und das Ventil 10 in der Frischluftleitung 28 durch die Steuereinrichtung 30 geschlossen. Nach Beenden der Kompostierung kann der Fermenter 2-2 geöffnet, die verbrauchte Biomasse entnommen und frische Biomasse eingebracht werden.

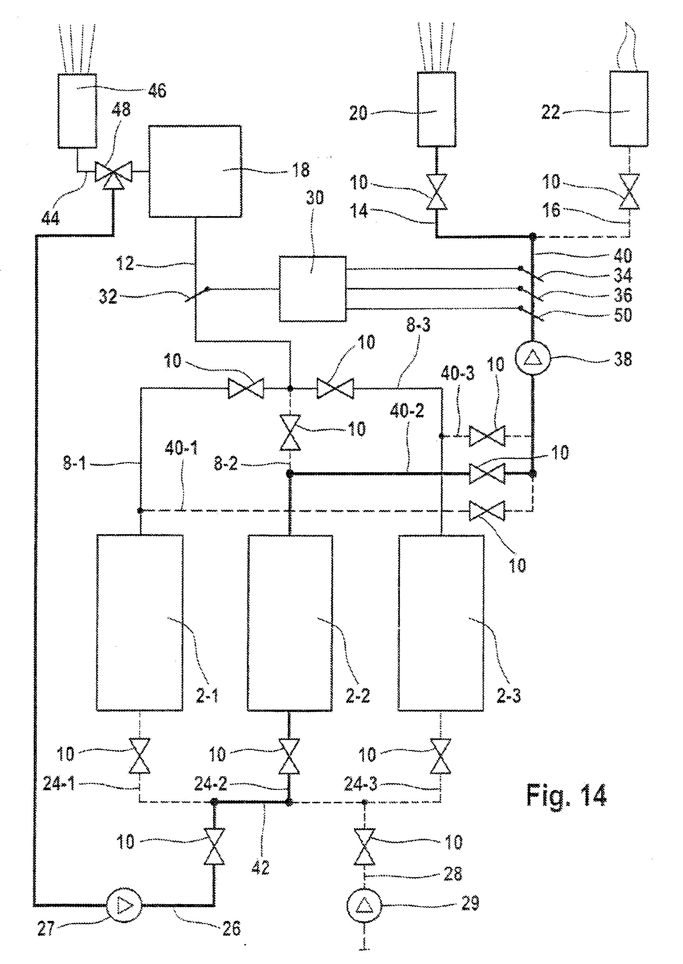

Ist der Fermenter 2-2 wieder mit frischer Biomasse beladen, wird die Be- und Entladeöffnung geschlossen, die Verbindung zwischen zweiten Biogasauslass 8-2 und Abgaskamin 20 über die zweite Teil-Biogas/Abgas-Leitung 40-2, die gemeinsame Biogas/Abgas-Leitung und die erste Biogas/Abgas-Leitung 14 wird aufrecht erhalten, und die Steuereinrichtung 30 öffnet das Ventil 10 in der Abgasleitung 26 und schaltet das 3-Wege-Ventil 48 in der Auspuffleitung 44 des BHKW 18, so das kohlendioxidhaltiges Abgas in den Fermenter 2-2 gepumpt wird - siehe Fig. 14. Dies wird solange fortgesetzt, bis die durch den zweiten Messfühler 34 erfasste Kohlendioxidkonzentration in der gemeinsamen Biogas/Abgas-Leitung 40 einen zweiten Grenzwert erreicht bzw. übersteigt.

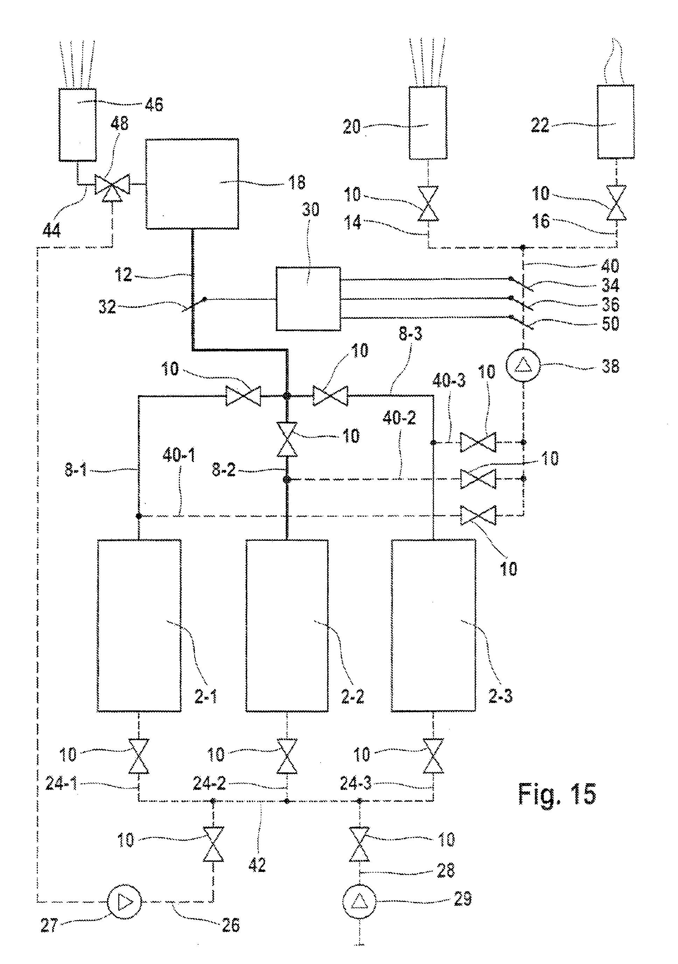

Ist dieser zweite Grenzwert für die Kohlendioxidkonzentration erreicht, wird durch die Steuereinrichtung 30 das Ventil 10 in der Abgasleitung 26 geschlossen, das 3-Wege-Ventil 38 geschalten, das Ventil 10 in der zweiten Teil-Biogas/AbgasLeitung 40-2 wird geschlossen, und das Ventil 10 in dem zweiten Biogasauslass 8-2 geöffnet, wie dies in Fig. 15 dargestellt ist. Damit hat auch der zweite Fermenter 2-2 wieder die Phase der Biogasproduktion erreicht, und das in dem Fermenter 2-2 erzeugte Biogas wird über die Biogasleitung 12 der Gasaufbereitungseinrichtung 44 und dem BHKW 18 zugeführt. Die Zuschaltung des Biogasauslasses 8-2 zu der gemeinsamen Biogasleitung 12 erfolgt erst dann, wenn die durch den vierten Messfühler 50 erfasste Methankonzentration einen vierten Grenzwert erreicht. Dieser vierte Grenzwert stimmt mit dem oberen Grenzwert überein.

Das Ventil 10 in der Abgasleitung 26 kann entfallen, da dessen Funktion auch durch das 3-Wege-Ventil 48 übernommen werden kann.

Nachfolgend sind beispielhafte Zahlenwerte für die verschiedenen Grenzwerte angegeben:

| | | | | |
|---|---|---|---|---|
| Methankonzentration: | oberer Grenzwert | 30% | bis | 50% |
| | mittlerer Grenzwert | 10% | bis | 20% |
| | unterer Grenzwert | 0% | bis | 3% |
| | vierter Grenzwert | 30% | bis | 50% |
| | | | | |
| Kohlendioxidkonzentration: | erster Grenzwert | 0,5% | bis | 2% |
| | zweiter Grenzwert | 5% | bis | 15% |

Der Abgasvolumenstrom in der Abgasleitung 26 beträgt je nach Größe der Fermenter und der Menge des zur Verfügung stehenden Abgases zwischen 150 und 1000 m³/h. Der Frischluftvolumenstrom in der Frischluftleitung 28 beträgt zwischen 1000 und 5000 m³/h.

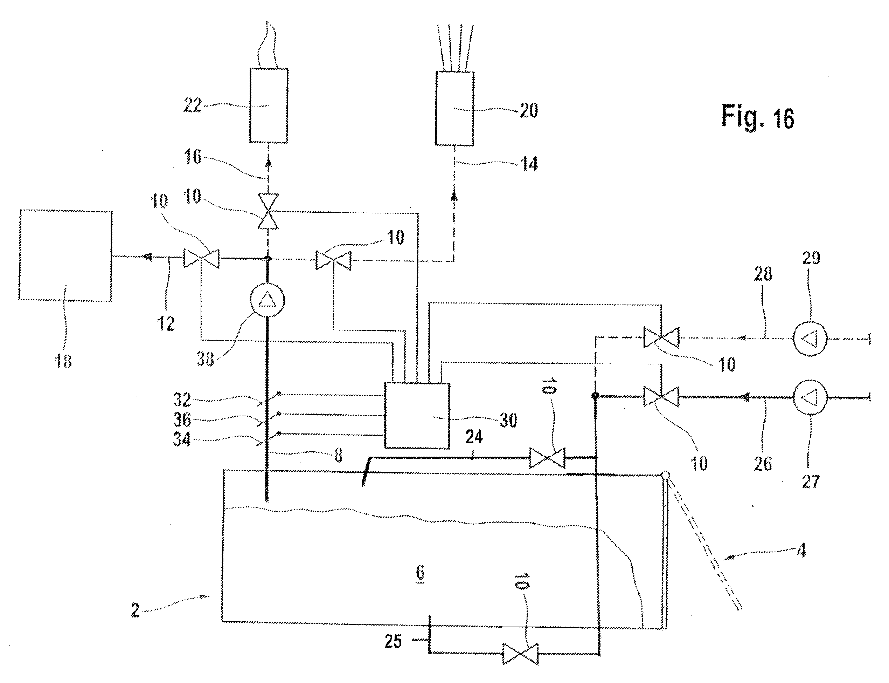

Fig. 16 zeigt eine Fig. 1 entsprechende Darstellung einer kombinierten Anlage gemäß einer Ausführungsform der Erfindung, die sich von der ersten Ausführungsform nach Fig. 1 bis 7 dadurch unterscheidet, dass das Spülgas in Form von Abgas oder Frischluft in den verschiedenen Betriebszuständen nicht nur über den ersten Spülgaseinlass 24 im Bereich über der Biomasse 6, sondern zusätzlich oder alternativ über einen zweiten Spülgaseinlass 25 im Bereich der Bodenplatte des Fermenters 2 zugeführt wird. Hierdurch wird erreicht, dass auch in der Biomasse 6 befindliches Biogas sicher "ausgespült" wird. Hierdurch wird zudem erreicht, dass der Methanschlupf während des Be- und Entladens des Fermenters weiter vermindert wird.

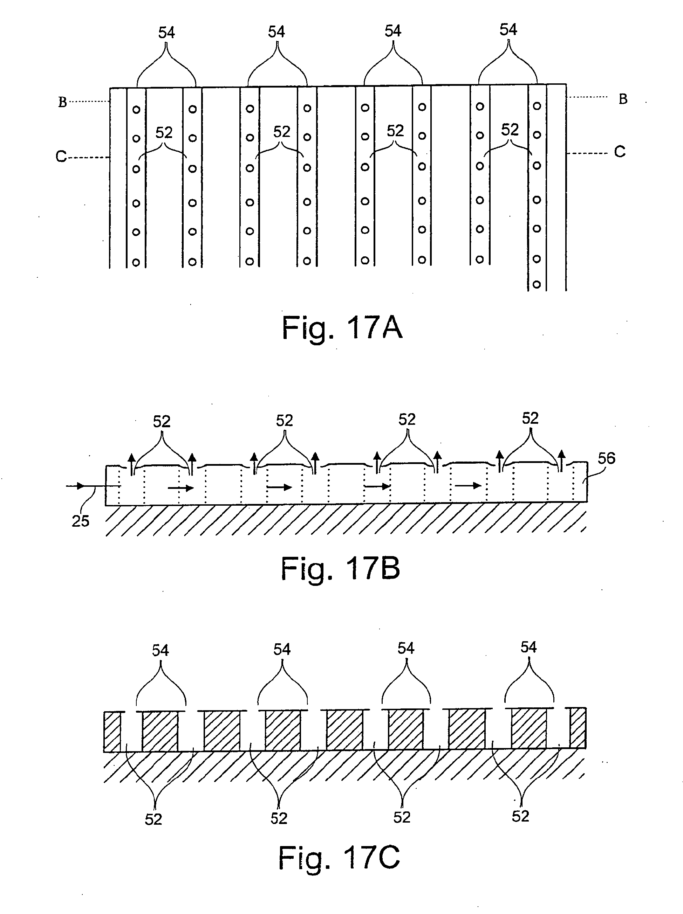

Fig. 17A zeigt eine Aufsicht auf die Bodenplatte des Fermenters 2 in der Ausführungsform nach Fig. 16. In der Bodenplatte des Fermenters 2 sind in Längsrichtung Spülgaskanäle 52 vorgesehen, die durch einen flüssigkeits- und gasdurchlässigen Rost 54 abgedeckt sind. Die verschiedenen, parallel verlaufenden Spülgaskanäle 52 werden an einer oder an mehreren Stellen durch einen quer zur Längsrichtung der Spülgaskanäle 52 verlaufenden Querkanal 56 miteinander verbunden. In diesen Querkanal 56 mündet der zweite Spülgaseinlass 25. Fig. 17B zeigt eine Schnittdarstellung entlang der Line B - B in Fig. 17A mit dem Querkanal 56 und den Spülgaskanälen 52. Fig. 17C zeigt eine Schnittdarstellung entlang der Line C - C in Fig. 17A mit den Spülgaskanälen 52.

Auch bei den Ausführungsformen nach den Fig. 8 und 9 bis 15 kann die Zuführung von Spülgas über Spülgaskanäle 52 im Boden des Fermenters 2 erfolgen. Bei den verschiedenen Ausführungsformen ist die Be- und Entladeöffnung jeweils auf der linken Seite des Fermenters 2 vorgesehen. Die Be- und Entladeöffnung kann auch auf der gegenüberliegenden Seite vorgesehen werden.

### Bezugszeichenliste

- 2: Fermenter
- 4: Be- und Entladeöffnung
- 6: Biomasse
- 8: Biogasauslass
- 10: Ventile
- 12: Biogasleitung
- 14: erste Biogas/Abgas-Leitung
- 16: zweite Biogas/Abgas-Leitung
- 18: Blockheizkraftwerk
- 20: Abgaskamin
- 22: Abgasfackel
- 24: Spülgaseinlass
- 25: zweiter Spülgaseinlass
- 26: Abgasleitung
- 27: Abgasgebläse
- 28: Frischluftleitung
- 29: Frischluftgebläse
- 30: Steuereinrichtung
- 32: erster Messfühler (Methankonzentration)
- 34: zweiter Messfühler (Kohlendioxidkonzentration)
- 36: dritter Messfühler (Volumenstrom)
- 38: Gebläse
- 40: gemeinsame Biogas/Abgas-Leitung
- 40-1: erste Teil-Biogas/Abgas-Leitung
- 40-2: zweite Teil-Biogas/Abgas-Leitung
- 40-3: dritte Teil-Biogas/Abgas-Leitung
- 42: gemeinsamer Spülgaseinlass
- 44: Auspuffleitung
- 46: zweiter Abgaskamin
- 48: 3-Wege-Ventil
- 50: vierter Messfühler (Methankonzentration)
- 52: Spülgaskanäle
- 54: Abdeckrost
- 56: Querkanal

## Patentansprüche

1. Kombinierte Anlage zur Erzeugung von Biogas und Kompost, mit wenigstens einem nach dem Prinzip der Trockenfermentation arbeitenden Fermenter (2) zur Erzeugung von Biogas im Batch-Betrieb mit einer Bodenplatte in der Spülgaskanäle (52) vorgesehen sind, einem Biogasauslass (8), einem ersten Spülgaseinlass (24) und einem zweiten Spülgaseinlass (25), der mit den Spülgaskanälen (52) verbunden ist;
einer Biogasleitung (12) die mit dem Biogasauslass (8) verbindbar ist;
einer Abgasleitung (26) mittels der kohlendioxidhaltiges Abgas dem wenigstens einen Spülgaseinlass (24, 25) zuführbar ist;
einem Abgaskamin (20), der über eine erste Biogas/Abgas-Leitung (14) mit dem Biogasauslass (8) verbindbar ist;
4e einer Abgasfackel (22), die über eine zweite Biogas/AbgasLeitung (16) mit dem Biogasauslass (8) verbindbar ist;
einer Frischluftleitung (28), die mit dem wenigstens einen Spülgaseinlass (24, 25) verbindbar ist;
einer Steuereinrichtung (30) zur Verbindung des Biogasauslasses (8) mit der Biogasleitung (12) oder mit dem Bioabgaskamin (20) über die erste Biogas/Abgas-Leitung (14) oder mit der Abgasfackel (22) über die zweite Biogas/Abgas-Leitung (16) und zur Verbindung des Spülgaseinlasses (24, 25) mit der Abgasleitung (26) oder mit der Frischluftleitung (28); und
einer Messeinrichtung (32, 34), die mit der Steuereinrichtung (30) verbunden ist und einen ersten Messfühler (32) zur Erfassung der Methankonzentration und einen zweiten Messfühler (34) zur Erfassung der Kohlendioxidkonzentration in dem aus dem wenigstens einen Fermenter (2) austretenden Gasgemisch aufweist.

2. Kombinierte Anlage nach Anspruch 1, **dadurch gekennzeichnet, dass** die Messeinrichtung (32, 34) in dem Biogasauslass (8) angeordnet ist.

3. Kombinierte Anlage nach Anspruch 1 oder 2, **dadurch gekennzeichnet, dass** eine Mehrzahl von Fermentern (2-i) vorgesehen sind, deren Biogasauslässe (8-i) in die gemeinsamen Biogasleitung (12) münden und dass der erste Messfühler zur Erfassung der Methankonzentration in der gemeinsamen Biogasleitung angeordnet ist.

4. Kombinierte Anlage nach Anspruch 3, **dadurch gekennzeichnet, dass** die Biogasauslässe (8-i) selektiv über eine gemeinsame Biogas/Abgas-Leitung (40) mit dem Abgaskamin (20) oder der Abgasfackel (22) verbindbar sind und dass der zweite Messfühler zur Erfassung der Kohlendioxidkonzentration in der gemeinsamen Biogas/Abgas-Leitung (40) angeordnet ist.

5. Kombinierte Anlage nach einem der vorhergehenden Ansprüche,
**dadurch gekennzeichnet, dass** die Abgasleitung (26) Abgas aus einer Verbrennungskraftmaschine zuführt.

6. Kombinierte Anlage nach einem der vorhergehenden Ansprüche,
**dadurch gekennzeichnet, dass** die Biogasleitung (12) die Verbindung zu einer Biogasverwertungseinrichtung herstellt, die kohlendioxidhaltiges Abgas erzeugt.

7. Kombinierte Anlage nach Anspruch 5, **dadurch gekennzeichnet, dass** die Biogasverwertungseinrichtung ein Blockheizkraftwerk (18) aufweist.

8. Kombinierte Anlage nach Anspruch 5, **dadurch gekennzeichnet, dass** die Biogasverwertungseinrichtung eine Brennstoffzelle aufweist.

9. Kombinierte Anlage nach Anspruch 5, **dadurch gekennzeichnet, dass** die Biogasverwertungseinrichtung eine Gasaufbereitungseinrichtung (44) aufweist.

10. Kombinierte Anlage nach einem der vorhergehenden Ansprüche,
**dadurch gekennzeichnet, dass** der erste Spülgaseinlass (24) im Bereich über der Biomasse (6) in den Fermenter (2) mündet.

11. Kombinierte Anlage nach einen der vorhergehenden Ansprüche, **dadurch gekennzeichnet, dass** die Spülgaskanäle (52) zur Abführung von aus der Biomasse während der Erzeugung von Biogas austretenden Sickerflüssigkeiten ausgelegt sind.

12. Verfahren zum Umschalten eines Fermenters in einer kombinierten Anlage nach einem der vorhergehenden Ansprüche zwischen Biogaserzeugung und Kompostierung mit den Verfahrensschritten:
a) Beibehalten der Verbindung zwischen Biogasauslass (8) und Biogasleitung (12);
b) Verbinden der Abgasleitung (26) mit dem wenigstens einen Spülgaseinlass (24, 25) des umzuschaltenden Fermenters (2);
c) Spülen des umzuschaltenden Fermenters (2) mit Abgas aus der Abgasleitung (26) bis die durch den ersten Messfühler (32) erfasste Methankonzentration auf einen oberen Grenzwert abgesunken ist;
d) Trennen der Biogasleitung (12) von dem Biogasauslass (8) des umzuschaltenden Fermenters (2);
e) Verbinden des Biogasauslasses (8) des umzuschaltenden Fermenters (2) mit der ersten Biogas/Abgas-Leitung (14) und Zufuhr des Abgas/Biogas-Gemisches zu dem Bioabgaskamin (20) bis die durch den ersten Messfühler (32) erfasste Methankonzentration auf einen unteren Grenzwert abgesunken ist;
f) Trennen der Abgasleitung (26) von dem Spülgaseinlass (24, 25) des umzuschaltenden Fermenters (2);
g) Verbinden der Frischluftleitung (28) mit dem Spülgaseinlass (24, 25) des umzuschaltenden Fermenters (2) und Zufuhr von Frischluft in den umzuschaltenden Fermenter (2) bis die durch den zweiten Messfühler (34) erfasste Kohlendioxidkonzentration auf einen ersten Grenzwert abgesunken ist; und
h) Kompostieren der verbrauchten Biomasse in dem Fermenter (2).

13. Verfahren nach Anspruch 12, **dadurch gekennzeichnet, dass** zwischen dem Verfahrensschritt d) und dem Verfahrensschritt e) folgende Verfahrensschritte ausgeführt werden:
d1) Verbinden des Biogasauslasses (8) des umzuschaltenden Fermenters (2) mit der zweiten Biogas/Abgas-Leitung (16) und Zufuhr des Abgas/Biogas-Gemisches zu der Abgasfackel (22) bis die durch den ersten Messfühler (32) erfasste Methankonzentration auf einen mittleren Grenzwert abgesunken ist, der zwischen dem oberen und dem unteren Grenzwert liegt; und
d2) Trennen des Biogasauslasses (8) des umzuschaltenden Fermenters (2) von der zweiten Biogas/Abgas-Leitung (16).

14. Verfahren nach Anspruch 12 oder 13, **dadurch gekennzeichnet, dass** die über die Frischluftleitung (28) zugeführte Frischluft vorgewärmt wird.

15. Verfahren nach einem der vorhergehenden Ansprüche 12 bis 14,
**dadurch gekennzeichnet, dass** der Kompostiervorgang durch die zugeführte Frischluftmenge gesteuert wird.

16. Verfahren nach einem der vorhergehenden Ansprüche 12 bis 15,
**dadurch gekennzeichnet, dass** die aus dem Fermenter (2) abgeführten Gasgemische gefiltert werden.

17. Verfahren zum Anfahren eines frisch mit Biomasse beladenen Fermenters (2) nach einem der vorhergehenden Ansprüche 1 bis 11 mit den Verfahrensschritten:
a) Schließen der Be- und Entladeöffnung (4);
b) Verbinden des Biogasauslasses (8) mit der ersten Biogas/Abgas-Leitung (14);
c) Verbinden der Abgasleitung (26) mit dem Spülgaseinlass (24, 25) des anzufahrenden Fermenters (2) und Zufuhr von Abgas zu dem anzufahrenden Fermenter (2) bis die durch den zweiten Messfühler (34) erfasste Kohlendioxidkonzentration einen zweiten Grenzwertwert erreicht;
d) Trennen der Abgasleitung (26) von dem Spülgaseinlass (24, 25);
e) Trennen der ersten Biogas/Abgas-Leitung (14) von dem Biogasauslass (8);
f) Verbinden der Biogasleitung (12) mit dem Biogasauslass (8).

18. Verfahren nach Anspruch 17, **dadurch gekennzeichnet, dass** der Verfahrensschritt f) ausgeführt wird, wenn die durch den ersten oder vierten Messfühler (32; 50) erfasste Methankonzentration einen vierten Grenzwert überschreitet.

19. Verfahren nach Anspruch 18, **dadurch gekennzeichnet, dass** der vierte Grenzwert der Methankonzentration gleich dem oberen Grenzwert der Methankonzentration ist.

20. Verfahren nach einem der vorhergehenden Ansprüche 12 bis 19, **dadurch gekennzeichnet, dass** die Abgasleitung (26) mit dem Auspuff einer Verbrennungskraftmaschine verbunden wird.

21. Verfahren nach einem der vorhergehenden Ansprüche 12 bis 19, **dadurch gekennzeichnet, dass** die Abgasleitung (26) mit dem Auspuff (48) einer kohlendioxidhaltiges Abgas erzeugenden Biogasaufbereitungsseinrichtung (44) verbunden ist.

22. Verfahren nach einem der vorhergehenden Ansprüche 12 bis 19, **dadurch gekennzeichnet, dass** die Abgasleitung (26) mit dem Auspuff einer Brennstoffzelle verbunden ist.

## Claims

1. Combined installation for the production of biogas and compost, including at least one fermenter (2) operating according to the principle of dry fermentation for the production of biogas in the batch mode and including a floor plate in which purging gas passages (52) are provided, a biogas outlet (8), a first purging gas inlet (24), and a second purging gas inlet (25) connected to the purging gas passages (52);
a biogas line (12) adapted to be connected to the biogas outlet (8);
a waste gas line (26) whereby waste gas containing carbon dioxide may be supplied to the at least one purging gas inlet (24, 25);
a waste gas chimney (20) adapted to be connected to the biogas outlet (8) via a first biogas/waste gas line (14);
a waste gas flare (22) adapted to be connected to the biogas outlet (8) via a second biogas/waste gas line (16);
a fresh air line (28) adapted to be connected to the at least one purging gas inlet (24, 25);
a control means (30) for connecting the biogas outlet (8) to the biogas line (12) or to the biogas/waste gas chimney (20) via the first biogas/waste gas line (14) or to the waste gas flare (22) via the second biogas/waste gas line (16) and for connecting the purging gas inlet (24, 25) to the waste gas line (26) or to the fresh air line (28); and
a measurement means (32, 34) connected to the control means (30) and including a first measurement sensor (32) for detecting the methane concentration and a second measurement sensor (34) for detecting the carbon dioxide concentration in the gas mixture emerging from the at least one fermenter (2).

2. Combined installation according to claim 1, **characterized in that** the measurement means (32, 34) is arranged in the biogas outlet (8).

3. Combined installation according to claim 1 or 2, **characterized in that** a plurality of fermenters (2-i) are provided, the biogas outlets (8-i) of which open into the common biogas line (12), and **in that** the first measurement sensor for detecting the methane concentration is arranged in the common biogas line.

4. Combined installation according to claim 3, **characterized in that** the biogas outlets (8-i) are adapted to be selectively connected to the waste gas chimney (20) or to the waste gas flare (22) via a common biogas/waste gas line (40), and **in that** the second measurement sensor for detecting the carbon dioxide concentration is arranged in the common biogas/waste gas line (40).

5. Combined installation according to any one of the preceding claims, **characterized in that** the waste gas line (26) supplies waste gas from an internal combustion engine.

6. Combined installation according to any one of the preceding claims, **characterized in that** the biogas line (12) establishes the connection with a biogas-utilising means producing waste gas that contains carbon dioxide.

7. Combined installation according to claim 5, **characterized in that** the biogas-utilising means includes a block-type thermal power station (18).

8. Combined installation according to claim 5, **characterized in that** the biogas-utilising means includes a fuel cell.

9. Combined installation according to claim 5, **characterized in that** the biogas-utilising means includes a gas processing means (44).

10. Combined installation according to any one of the preceding claims, **characterized in that** the first purging gas inlet (24) opens into the fermenter (2) in the area above the biomass (6).

11. Combined installation according to any one of the preceding claims, **characterized in that** the purging gas passages (52) are adapted to discharge seepage liquids seeping from the biomass during the production of biogas.

12. Method of switching a fermenter in a combined installation according to any one of the preceding claims between biogas production and composting, including the method steps of:
a) maintaining the connection between biogas outlet (8) and biogas line (12);
b) connecting the waste gas line (26) to the at least one purging gas inlet (24, 25) of the fermenter (2) to be switched over;
c) purging the fermenter (2) to be switched over with waste gas from the waste gas line (26) until the methane concentration detected by the first measurement sensor (32) has dropped to an upper limit;
d) disconnecting the biogas line (12) from the biogas outlet (8) of the fermenter (2) to be switched over;
e) connecting the biogas outlet (8) of the fermenter (2) to be switched over to the first biogas/waste gas line (14) and supplying the waste gas/biogas mixture to the biogas/waste gas chimney (20) until the methane concentration detected by the first measurement sensor (32) has dropped to a lower limit;
f) disconnecting the waste gas line (26) from the purging gas inlet (24, 25) of the fermenter (2) to be switched over;
g) connecting the fresh air line (28) to the purging gas inlet (24, 25) of the fermenter (2) to be switched over and supplying fresh air into the fermenter (2) to be switched over until the carbon dioxide concentration detected by the second measurement sensor (34) has dropped to a first limit; and
h) composting the spent biomass inside the fermenter (2).

13. Method according to claim 12, **characterized in that** the following method steps are carried out between method step d) and method step e):
d1) connecting the biogas outlet (8) of the fermenter (2) to be switched over to the second biogas/waste gas line (16) and supplying the waste gas/biogas mixture to the waste gas flare (22) until the methane concentration detected by the first measurement sensor (32) has dropped to a medium limit that is situated between the upper and lower limits; and
d2) disconnecting the biogas outlet (8) of the fermenter (2) to be switched over from the second biogas/waste gas line (16).

14. Method according to claim 12 or 13, **characterized in that** the fresh air supplied via the fresh air line (28) is pre-heated.

15. Method according to any one of the preceding claims 12 to 14, **characterized in that** the composting process is controlled by way of the supplied amount of fresh air.

16. Method according to any one of the preceding claims 12 to 15, **characterized in that** the gas mixtures discharged from the fermenter (2) are filtered.

17. Method for starting up a fermenter (2) according to any one of the preceding claims 1 to 11 that was freshly charged with biomass, including the following method steps:
a) closing the loading and unloading opening (4);
b) connecting the biogas outlet (8) to the first biogas/waste gas line (14);
c) connecting the waste gas line (26) to the purging gas inlet (24, 25) of the fermenter (2) to be started up, and supplying waste gas to the fermenter (2) to be started up, until the carbon dioxide concentration detected by the second measurement sensor (34) has reached a second limit;
d) disconnecting the waste gas line (26) from the purging gas inlet (24, 25);
e) disconnecting the first biogas/waste gas line (14) from the biogas outlet (8);
f) connecting the biogas line (12) to the biogas outlet (8).

18. Method according to claim 17, **characterized in that** method step f) is carried out when the methane concentration detected by the first or fourth measurement sensor (32; 50) exceeds a fourth limit.

19. Method according to claim 18, **characterized in that** the fourth limit of methane concentration is equal to the upper limit of methane concentration.

20. Method according to any one of the preceding claims 12 to 19, **characterized in that** the waste gas line (26) is connected to the exhaust of an internal combustion engine.

21. Method according to any one of the preceding claims 12 to 19, **characterized in that** the waste gas line (26) is connected to the exhaust (48) of a biogas processing means (44) that produces waste gas containing carbon dioxide.

22. Method according to any one of the preceding claims 12 to 19, **characterized in that** the waste gas line (26) is connected to the exhaust of a fuel cell.

## Revendications

1. Installation combinée de production de biogaz et de compost, comprenant au moins un fermenteur (2) fonctionnant selon le principe de la fermentation sèche pour produire du biogaz en mode discontinu et pourvu d'une plaque de fond dans laquelle sont prévus des canaux de gaz de purge (52), une sortie de biogaz (8), une première admission de gaz de purge (24) et une seconde admission de gaz de purge (25), laquelle est raccordée aux canaux de gaz de purge (52) ;
un conduit de biogaz (12) pouvant être raccordé à la sortie de biogaz (8) ;
un conduit d'échappement (26) par lequel des gaz d'échappement contenant du dioxyde de carbone peuvent être amenés à ladite admission de gaz de purge (24, 25) au moins au nombre de une ;
une cheminée d'échappement des gaz (20) pouvant être raccordée à la sortie de biogaz (8) par l'intermédiaire d'un premier conduit de biogaz/gaz d'échappement (14) ;
une torchère (22) pouvant être raccordée à la sortie de biogaz (8) par l'intermédiaire d'un second conduit de biogaz/gaz d'échappement (16) ;
un conduit d'air frais (28) pouvant être raccordé à ladite admission de gaz de purge (24, 25) au moins au nombre de une ;
un dispositif de commande (30) pour raccorder la sortie de biogaz (8) au conduit de biogaz (12) ou à la cheminée d'échappement de biogaz (20) par l'intermédiaire du premier conduit de biogaz/gaz d'échappement (14) ou à la torchère (22) par l'intermédiaire du second conduit de biogaz/gaz d'échappement (16), et pour raccorder l'admission de gaz de purge (24, 25) au conduit d'échappement (26) ou au conduit d'air frais (28) ; et
un dispositif de mesure (32, 34) qui est relié au dispositif de commande (30) et comprend un premier capteur de mesure (32) pour détecter la concentration de méthane et un second capteur de mesure (34) pour détecter la concentration de dioxyde de carbone dans le mélange gazeux sortant dudit fermenteur (2) au moins au nombre de un.

2. Installation combinée selon la revendication 1, **caractérisée en ce que** le dispositif de mesure (32, 34) est disposé dans la sortie de biogaz (8).

3. Installation combinée selon la revendication 1 ou 2, **caractérisée en ce qu'**est prévue une pluralité de fermenteurs (2-i) dont les sorties de biogaz (8-i) débouchent dans le conduit de biogaz commun (12) et **en ce que** le premier capteur de mesure pour la détection de la concentration de méthane est disposé dans le conduit de biogaz commun.

4. Installation combinée selon la revendication 3, **caractérisée en ce que** les sorties de biogaz (8-i) peuvent être raccordées de manière sélective à la cheminée d'échappement des gaz (20) ou à la torchère (22) par l'intermédiaire d'un conduit de biogaz/gaz d'échappement (40) commun, et **en ce que** le second capteur de mesure pour détecter la concentration de dioxyde de carbone est disposé dans le conduit de biogaz/gaz d'échappement (40) commun.

5. Installation combinée selon l'une des revendications précédentes, **caractérisée en ce que** le conduit d'échappement (26) achemine les gaz d'échappement d'un moteur à combustion interne.

6. Installation combinée selon l'une des revendications précédentes, **caractérisée en ce que** le conduit de biogaz (12) permet la liaison avec un dispositif de valorisation de biogaz qui génère des gaz d'échappement contenant du dioxyde de carbone.

7. Installation combinée selon la revendication 5, **caractérisée en ce que** le dispositif de valorisation de biogaz comprend une centrale de cogénération (18).

8. Installation combinée selon la revendication 5, **caractérisée en ce que** le dispositif de valorisation de biogaz comprend une pile à combustible.

9. Installation combinée selon la revendication 5, **caractérisée en ce que** le dispositif de valorisation de biogaz comprend un dispositif de traitement de gaz (44).

10. Installation combinée selon l'une des revendications précédentes, **caractérisée en ce que** la première admission de gaz de purge (24) dans la région située au-dessus de la biomasse (6) débouche dans le fermenteur (2).

11. Installation combinée selon l'une des revendications précédentes, **caractérisée en ce que** les canaux de gaz de purge (52) sont conçus pour évacuer des liquides de suintement sortant de la biomasse pendant la production de biogaz.

12. Procédé pour la commutation d'un fermenteur, dans une installation combinée selon l'une des revendications précédentes, entre la production de biogaz et le compostage, comprenant les étapes suivantes :
a) maintenir la liaison entre la sortie de biogaz (8) et le conduit de biogaz (12) ;
b) raccorder le conduit d'échappement (26) à ladite admission de gaz de purge (24, 25) au moins au nombre de une du fermenteur (2) à commuter ;
c) rincer le fermenteur (2) à commuter avec du gaz d'échappement sortant du conduit d'échappement (26) jusqu'à ce que la concentration de méthane détectée par le premier capteur de mesure (32) chute à une valeur limite supérieure ;
d) déconnecter le conduit de biogaz (12) de la sortie de biogaz (8) du fermenteur (2) à commuter ;
e) raccorder la sortie de biogaz (8) du fermenteur (2) à commuter au premier conduit de biogaz/gaz d'échappement (14) et amener le mélange gaz d'échappement/biogaz à la cheminée d'échappement de biogaz (20) jusqu'à ce que la concentration de méthane détectée par le premier capteur de mesure (32) chute à une valeur limite inférieure ;
f) déconnecter le conduit d'échappement (26) de l'admission de gaz de purge (24, 25) du fermenteur (2) à commuter ;
g) raccorder le conduit d'air frais (28) à l'admission de gaz de purge (24, 25) du fermenteur (2) à commuter et amener de l'air frais au fermenteur (2) à commuter jusqu'à ce que la concentration de dioxyde de carbone détectée par le second capteur de mesure (34) chute à une première valeur limite ; et
h) composter la biomasse consommée dans le fermenteur (2).

13. Procédé selon la revendication 12, **caractérisé en ce qu'**entre l'étape d) et l'étape e) du procédé sont réalisées les étapes suivantes :
d1) raccorder la sortie de biogaz (8) du fermenteur (2) à commuter au second conduit de biogaz/gaz d'échappement (16) et amener le mélange gaz d'échappement/biogaz à la torchère (22) jusqu'à ce que la concentration de méthane détectée par le premier capteur de mesure (32) chute à une valeur limite moyenne située entre la valeur limite supérieure et la valeur limite inférieure ; et
d2) déconnecter la sortie de biogaz (8) du fermenteur (2) à commuter du second conduit de biogaz/gaz d'échappement (16).

14. Procédé selon la revendication 12 ou 13, **caractérisé en ce que** l'air frais alimenté via le conduit d'air frais (28) est préchauffé.

15. Procédé selon l'une des revendications précédentes 12 à 14, **caractérisé en ce que** le processus de compostage est commandé par la quantité d'air frais alimenté.

16. Procédé selon l'une des revendications précédentes 12 à 15, **caractérisé en ce que** les mélanges gazeux évacués du fermenteur (2) sont filtrés.

17. Procédé pour démarrer un fermenteur (2) fraîchement chargé de biomasse selon l'une des revendications précédentes 1 à 11, comprenant les étapes suivantes :
a) fermer l'ouverture de chargement et de déchargement (4) ;
b) raccorder la sortie de biogaz (8) au premier conduit de biogaz/gaz d'échappement (14) ;
c) raccorder le conduit d'échappement (26) à l'admission de gaz de purge (24, 25) du fermenteur (2) à démarrer et amener des gaz d'échappement au fermenteur (2) à démarrer jusqu'à ce que la concentration de dioxyde de carbone détectée par le second capteur de mesure (34) atteigne une seconde valeur limite ;
d) déconnecter le conduit d'échappement (26) de l'admission de gaz de purge (24, 25) ;
e) déconnecter le premier conduit de biogaz/gaz d'échappement (14) de la sortie de biogaz (8) ;
f) raccorder le conduit de biogaz (12) à la sortie de biogaz (8).

18. Procédé selon la revendication 17, **caractérisé en ce que** l'étape de procédé f) est réalisée lorsque la concentration de méthane détectée par le premier ou le quatrième capteur de mesure (32 ; 50) dépasse une quatrième valeur limite.

19. Procédé selon la revendication 18, **caractérisé en ce que** la quatrième valeur limite de la concentration de méthane est égale à la valeur limite supérieure de concentration de méthane.

20. Procédé selon l'une des revendications précédentes 12 à 19, **caractérisé en ce que** le conduit d'échappement (26) est raccordé à l'échappement d'un moteur à combustion interne.

21. Procédé selon l'une des revendications précédentes 12 à 19, **caractérisé en ce que** le conduit d'échappement (26) est raccordé à l'échappement (48) d'un dispositif de traitement de biogaz (44) générant des gaz d'échappement contenant du dioxyde de carbone.

22. Procédé selon l'une des revendications précédentes 12 à 19, **caractérisé en ce que** le conduit d'échappement (26) est raccordé à l'échappement d'une pile à combustible.
